# EUROPÄISCHE PATENTSCHRIFT

(11) **EP 2 292 334 B1**
(45) Veröffentlichungstag und Bekanntmachung des Hinweises auf die Patenterteilung: **30.11.2016**
(21) Anmeldenummer: 10175296.2
(22) Anmeldetag: 03.09.2010
(51) Int. Cl.: B04B 5/04, B04B 13/00, B01L 3/14, B01L 3/00, A61M 1/36

(54) **Zentrifuge zum Trennen von Vollblut, kommunizierende Behälter dafür, sowie Verfahren zur Gewinnung eines hochangereichten Thrombozytenkonzentrats aus Vollblut**
Centrifuge for separating blood, fluidically communication containers therefore and method for generating high concentration thrombocyte concentrate from blood
Centrifugeuse destinée à séparer du sang, récipients à communication fluidique correspondants, ainsi que procédé de production de concentré de thrombocytes hautement enrichis à partir de sang

(30) Priorität: 08.09.2009 DE 102009040525
(43) Veröffentlichungstag der Anmeldung: 09.03.2011
(73) Patentinhaber: Andreas Hettich GmbH & Co. KG, D-78532 Tuttlingen (DE)
(72) Erfinder: Eberle, Klaus-Günter, 78532 Tuttlingen (DE)
(74) Vertreter: Puschmann Borchert Bardehle Patentanwälte Partnerschaft mbB

(56) Entgegenhaltungen:
- DE-A1- 2 946 198
- US-A1- 2004 182 788
- US-A1- 2009 209 402

## Beschreibung

Die Erfindung betrifft eine Zentrifuge zum Trennen von Vollblut in seine Blutkomponenten sowie fluidisch kommunizierende Behälter zum Einsetzen in die Zentrifuge sowie ein Verfahren zur Gewinnung eines hochangereicherten Thrombozytenkonzentrats aus Vollblut mittels der Zentrifuge gemäß den Patentansprüchen 1,7 und 14.

Die Gewinnung eines hochangereicherten Thrombozytenkonzentrats aus Vollblut ist grundsätzlich bekannt. Hierzu wird mittels einer Zentrifuge in einem ersten Zentrifugationsvorgang das Vollblut in Erythrozyten mit höherer Dichte und thrombozyten- bzw. plättchenreiches Plasma getrennt. Eine Zwischenschicht aus Leukozyten bildet eine Trennschicht zwischen den Erythrozyten und dem thrombozyten- bzw. plättchenreichen Plasma. In einem anschließenden zweiten Zentrifugationsvorgang wird dann das thrombozyten- bzw. plättchenreiche Plasma in thrombozytenarmes Plasma und das gewünschte Thrombozytenkonzentrat getrennt.

Aus der EP 1 637 172 B1 ist ein Verfahren zum Trennen von in Beuteln befindlichen Blutkomponenten bekannt. Neben einer Trennung des Vollbluts in Erythrozyten und thrombozytenreiches Blutplasma ist mit der offenbarten Vorrichtung grundsätzlich eine Weiterverarbeitung des thrombozytenreichen Blutplasmas und damit die Gewinnung von Thrombozytenkonzentrat möglich. Neben einer Zentrifuge für das Zentrifugieren ist hierbei nach der Lehre von EP 1 637 172 B1 eine weitere Vorrichtung für das Abpressen vorgesehen. Der erforderliche Wechsel zwischen Zentrifuge einerseits und Abpressvorrichtung andererseits erweist sich als umständlich und zeitaufwendig.

Die US 2009/0209402 A1 offenbart eine Zentrifuge zum Trennen von Blutkomponenten unter Verwendung von mehreren, in der Zentrifuge angeordneten Behältern. Die Behälter weisen einen im Innern verschiebbaren Kolben auf und stehen über Leitungen, in denen Ventile angeordnet sind, im fluidischen Kontakt zueinander. Hierbei weisen die Kolben unterschiedliche Massen auf, so dass beim Zentrifugieren aufgrund der wirkenden Zentrifugalkraft Blutkomponenten von einem zu einem anderen Behälter verschoben werden können.

Ein weiteres Verfahren zum Trennen von in Blutbeuteln befindlichen Blutkomponenten mittels einer Zentrifuge ist aus der DE 29 46198 A1 bekannt. Das offenbarte Verfahren zeichnet sich insbesondere dadurch aus, dass die Verdrängung, d.h. das Auslassen der jeweils abzentrifugierten Komponenten unter Wirkung der Zentrifugalkraft während des Zentrifugierens erfolgt. Zusätzliche Verdrängungskörper im Blutbeutel oder eine Steuerung für die Expansion des Verdrängungskörpers sind nicht vorgesehen.

Der Erfindung liegt die Aufgabe zugrunde, eine Zentrifuge zur Verfügung zu stellen, die eine vereinfachte Herstellung eines hochangereichten Thrombozytenkonzentrats ermöglicht.

Diese Aufgabe wird durch die Merkmale des Patentanspruches 1 gelöst.

Die Unteransprüche 2 bis 6 stellen vorteilhafte Weiterbildungen der Erfindung dar.

Gemäß der Erfindung umfasst die Zentrifuge zum Trennen von Vollblut in seine Blutkomponenten eine Regel- und/oder Steuereinheit sowie eine mit der Regel- und/oder Steuereinheit gekoppelten Antriebseinheit, einen Rotor mit mindestens zwei Behälteraufnahmen zur lösbaren Halterung von fluidisch miteinander kommunizierender Behälter, sowie mindestens einen zwischen den Behälteraufnahmen angeordneten, mit der Regel- und/oder Steuereinheit gekoppelten Sensor zur Detektierung einer Trennschicht. Den Behälteraufnahmen ist jeweils eine mit der Regel- und/oder Steuereinheit gekoppelte Motor- und Getriebeeinheit zugeordnet, die jeweils über Mittel mit den in der jeweiligen Behälteraufnahme gehaltenen Behälter derart in Wirkverbindung stehen, dass eine Überführung und Rückführung von Blutkomponenten zwischen den Behältern initiierbar ist. In vorteilhafter Weise ist durch die erfindungsgemäße Zentrifuge nunmehr möglich eine automatisierte Überführung und Rückführung von Blutkomponenten zwischen den Behältern in der Zentrifuge durchzuführen. Da eine Entnahme eines der beiden Behälter nicht notwendig ist, ist eine unerwünschte Wiedervermischung ausgeschlossen, so dass die Herstellung einer hochangereichten Blutkomponente, insbesondere die Herstellung eines hochangereichten Thrombozytenkonzentrats, gewährleistet ist. Da, wie bereits ausgeführt, die Behälter während einer Überführung bzw. Rückführung von Blutkomponenten zwischen den Behältern in der Zentrifuge verbleiben, ist zudem eine vereinfachte Handhabung, eine verkürzte Bearbeitungsdauer, sowie eine reproduzierbare Gewinnung von Thrombozytenkonzentraten mit definierten, hochqualifizierten Eigenschaften sichergestellt.

Gemäß einer besonders vorteilhaften Ausführungsform der Erfindung weist der Rotor mindestens zwei, jeweils zwei Behälteraufnahmen aufweisende Behälteraufnahmepaare zur lösbaren Halterung von je zwei fluidisch miteinander kommunizierenden Behältem auf, wobei zwischen jedem Behälteraufnahmepaar ein Sensor zur Detektierung einer Trennschicht angeordnet ist. Die Behälteraufnahmepaare stellen jeweils geschlossene Systeme zur sterilen Prozessierung von Blutproben dar. Die paarweise Anordnung zweier Behälter erweist sich als vorteilhaft, da hierdurch eine optimale Auslastung der Zentrifuge und ein ruhiger Lauf für optimale Trennergebnisse gewährleistet ist.

Vorzugsweise ist dabei jedem Behälteraufnahmepaar ein weiterer Sensor zugeordnet, über den eine Bestückung eines Behälteraufnahmepaares mit Behältern detektierbar ist. Das Vorsehen des weiteren Sensors hat den positiven Effekt, dass eine Nichtbeladung eines Behälterpaares und damit eine Unwucht vorzeitig erkennbar ist.

Um eine kostengünstige und zuverlässige Detektion der Trennschicht bzw. der Bestückung eines Behälteraufnahmepaares zu gewährleisten, sind die Sensoren als optische Sensoren ausgebildet.

Zur Sicherstellung einer störungsarmen Daten- und Energieübertragung weist die Zentrifuge eine induktive Schnittstelle zur induktiven Daten- und Energieübertragung auf.

Der Erfindung liegt weiterhin die Aufgabe zugrunde, fluidisch kommunizierende Behälter für die Zentrifuge zur Verfügung zu stellen, die neben einer vereinfachten Handhabung auch einen guten Wirkungsgrad sicherstellen.

Diese Aufgabe wird durch die Merkmale des Anspruchs 7 gelöst.

Die Unteransprüche 8 bis 13 stellen vorteilhafte Weiterbildungen der fluidisch kommunizierenden Behälter dar.

Erfindungsgemäß bilden die fluidisch kommunizierenden Behälter ein geschlossenes System zur sterilen Verarbeitung von Blutproben und die Behälter sind jeweils in Form eines Röhrchens mit einem im Inneren des Röhrchens verfahrbar angeordneten Kolben ausgebildet, wobei die Röhrchen jeweils über eine lösbar mit dem Kolben in Kontakt stehende Schubstange in Wirkverbindung mit der der jeweiligen Behälteraufnahme zugeordneten Motor- und Getriebeeinheit stehen. Die erfindungsgemäße Ausbildung der Behälter erweist sich als besonders vorteilhaft, da die Röhrchen aufgrund ihrer festen Struktur keine Sedimentationsnester aufweisen und somit ein guter Wirkungsgrad sichergestellt ist. Da zudem aufgrund der in den Röhrchen angeordneten Kolben, die Kopplung mittels einer Schubstange mit der zugeordneten Motor- und Getriebeeinheit der Zentrifuge auf eine Art und Weise herstellbar ist, ist auch eine einfache Handhabung gewährleistet.

Gemäß einer besonders vorteilhaften Ausführungsform der Erfindung, ist bei mindestens einem Röhrchen der Kolben zweiteilig, in Einzugsrichtung des Kolbens betrachtet einen vorderen Kolbenabschnitt und einem hinteren Kolbenabschnitt aufweisend ausgebildet. Hierbei sind der vordere und hintere Kolbenabschnitt lösbar miteinander verbunden und der hintere Kolbenabschnitt weist einen mit einer Dichtung fluiddicht verschlossene Durchgangsbohrung auf, so dass nach Lösen der beiden Kolbenabschnitte der vordere Kolbenabschnitt mittels einer durch die Durchgangsbohrung im hinteren Kolbenabschnitt hindurchführbaren Kolbenstange relativ zum hinteren Kolbenabschnitt bewegbar ist. Die zweiteilige Ausführung des Kolbens erweist sich als besonders vorteilhaft, da hierdurch mittels einer Auf- und Abbewegung des vorderen Kolbenabschnitts eine gezielte durch Durchmischung der in diesen Bereich des Röhrchens befindlichen Blutkomponente ermöglicht ist.

Vorzugsweise sind hierbei der hintere und vordere Kolbenabschnitt mittels eines Bajonettverschlusses lösbar miteinander verbunden und der vordere Kolbenabschnitt weist eine Bohrung mit einem Innengewinde und die Kolbenstange in ihrem vorderen Bereich ein hierzu korrespondierend ausgebildetes Außengewinde auf. Die Ausbildung der lösbaren Verbindung mittels eines Bajonettverschlusses sowie das Vorsehen einer Schraubverbindung zwischen Kolbenstange und vorderen Kolbenabschnitt erweist sich als besonders vorteilhaft, da sowohl der Bajonettverschluss als auch die Verschraubung zwischen Kolbenstange und vorderem Kolbenabschnitt mittels einer Drehbewegung betätigbar sind. So ist beispielsweise die Kolbenstange mittels einer Drehung in Uhrzeigersinn mit dem vorderen Kolbenabschnitt verbindbar, während - nachdem die Kolbenstange mit dem vorderen Kolbenabschnitt fest verbunden ist - eine anschließende Drehung entgegen dem Uhrzeigersinn eine Lösung des Bajonettverschlusses bewirkt.

Um eine effektive Detektierung der Trennschicht mittels des optischen Sensors zu gewährleisten, sind die zwei Röhrchen mittels eines transparenten Schlauchs fluidisch kommunizierend miteinander verbunden.

Gemäß einer weiteren Ausführungsform sind drei oder mehr Röhrchens mittels eines transparenten Schlauchs, der über ein Kuppelelement in zwei oder mehr, jeweils ein Absperrventil aufweisende Schlauchstränge aufgeteilt ist, fluidisch kommunizierend miteinander verbunden. Ist beispielsweise ein Röhrchen mit Vollblut gefüllt und über einen ein Kuppelelement aufweisender Schlauch mit zwei weiteren Röhrchen fluidisch verbunden, ist nun in vorteilhafter Weise neben einer Trennung des Vollbluts in seine Blutkomponenten auch eine Überführung der getrennten Blutkomponenten in zwei separate Röhrchen ermöglicht.

Vorzugsweise weist der Schlauch ein integriertes Ventil auf. Die Integration eines Ventils hat den Effekt, dass hierdurch ein ungewolltes Rückfließen während der Zentrifugation bzw. nach einem Zentrifugationsvorgang verhindert wird.

Gemäß einer weiteren vorteilhaften Ausführungsform der Erfindung weist der Schlauch an seinen Enden Befestigungselemente auf, über die der Schlauch lösbar über die Verschlusskappen der Röhrchen befestigbar ist. Das Vorsehen der Befestigungselemente erweist sich als vorteilhaft, da hierdurch eine einfache Handhabung sicher gestellt ist.

Der Erfindung liegt weiterhin die Aufgabe zugrunde, ein Verfahren zur Gewinnung eines hochangereicherten Thrombozytenkonzentrats aus Vollblut zur Verfügung zu stellen.

Diese Aufgabe wird durch die Merkmale des Patentanspruches 14 gelöst.

Die Unteransprüche 15 und 16 stellen eine vorteilhafte Weiterbildung des Verfahrens dar.

Das erfindungsgemäße Verfahren umfasst die nachfolgenden Schritte:
- Einlegen mindestens zweier über einen Schlauch fluidisch miteinander verbundene Röhrchen in die Behälteraufnahmen, wobei das erste Röhrchen das Vollblut und das zweite Röhrchen, nachfolgend auch als Sekundärröhrchen bezeichnet, gegebenenfalls einen biokompatiblen Werkstoff enthält;
- Herstellung einer Wirkverbindung zwischen den in den Röhrchen befindlichen Kolben und den zugeordneten Motor- und Getriebeinheiten mittels einer Schubstange;
- Anlaufen der Zentrifuge auf eine erste Zentrifugendrehzahl;
- Erstes Zentrifugieren, bis sich im ersten Röhrchen Erythrozytenkonzentrat mit höherer Dichte sowie durch eine Trennschicht davon separiertes thrombozytenreiches Blutplasma ausgebildet haben,
- Reduzieren der Zentrifugendrehzahl auf eine erste Abpressdrehzahl;
- Anlaufen der dem ersten Röhrchen zugeordneten und mit dem Kolben in Wirkverbindung stehenden Motor- und Getriebeeinheit und Auspressen des thrombozytenreichen Blutplasmas über den Schlauch in das Sekundärröhrchen bis der Sensor die Trennschicht detektiert hat;
- Erhöhen der Drehzahl auf eine zweite Zentrifugendrehzahl;
- Zweites Zentrifugieren bis sich im Sekundärröhrchen ein Thrombozytenkonzentrat mit höherer Dichte sowie separiertes thrombozytenarmes Blutplasma ausgebildet haben;
- Reduzieren der Zentrifugendrehzahl auf eine zweite Abpressdrehzahl;
- Anlaufen der dem Sekundärröhrchen zugeordneten und mit dem Kolben in Wirkverbindung stehenden Motor- und Getriebeeinheit und Rückpressung des thrombozytenarmen Blutplasmas in das erste Röhrchen;
- Abbremsen der Zentrifuge;
- Zurückfahren der Motor- und Getriebeeinheiten;
- Herausnahme der Röhrchen.

Da gemäß dem erfindungsgemäßen Verfahren das thrombozytenarme Blutplasma vor Entnahme des Sekundärröhrchens aus dem Sekundärröhrchen entfernt wird, ist eine Wiedervermischung ausgeschlossen, sodass in vorteilhafter Weise durch das erfindungsgemäße Verfahren ein hochangereichertes Thrombozytenkonzentrat herstellbar ist.

Vorzugsweise werden dabei mit Ausnahme der Schritte Einlegen und Entnahme der Röhrchen sämtliche Verfahrensschritte automatisiert d.h. gemäß einem vorher festlegbaren Ablaufschema durchgeführt. Da die Parameter für das erste Zentrifugieren (Zeit, Drehzahl oder RCF/Radius, Anlauf- und Bremsrampen), die erste Abpressdrehzahl, die Parameter für das zweite Zentrifugieren (Zeit, Drehzahl oder RCF/Radius, Anlauf- und Bremsrampen), die zweite Abpressdrehzahl, das Rückpressvolumen beim Rückpressvorgang, sowie die Bremsrampe zum Abbremsen der Zentrifuge, nach einem vorher festgelegten Ablaufschema durchgeführt werden, ist eine Fehlbedienung seitens des Bedienpersonals nahezu ausgeschlossen.

Obige Parameter können dabei in bequemer Art und Weise durch das Bedienpersonal bei stehender Zentrifuge in der Regel- und/oder Steuereinheit eingegeben bzw. geändert und an die jeweilige Applikation angepasst werden.

Insbesondere für den Fall, dass das Sekundärröhrchen einen biokompatiblen Werkstoff enthalten hat, wird gemäß einer besonders vorteilhaften Ausführungsform bei dem Verfahren zur Herstellung des Thrombozytenkonzentrats als Sekundärröhrchen ein Röhrchen mit einem zweigeteilten Kolben verwendet und es wird nach Herausnahme des Sekundärröhrchens aus der Zentrifuge der Inhalt des Sekundärröhrchens, d.h. biokompatibler Werkstoff und Thrombozytenkonzentrat, mittels des vorderen Kolbenabschnitts durchmischt. Dies hat den Effekt, dass, wenn es die Applikation erfordert, eine homogene Paste aus Thrombozytenkonzentrat und biokompatiblem Werkstoff zum Auffüllen von Defekten gewonnen werden kann. Diese wird im Anschluss in den Defekt appliziert.

Weitere Vorteile, Merkmale und Anwendungsmöglichkeiten der vorliegenden Erfindung ergeben sich aus der nachfolgenden Beschreibung in Verbindung mit dem in der Zeichnung dargestellten Ausführungsbeispiel.

Die Erfindung wird im Folgenden anhand des in der Zeichnung dargestellten Ausführungsbeispiels näher beschrieben. In der Beschreibung, in den Ansprüchen und in der Zeichnung werden die in der unten aufgeführten Liste der Bezugszeichen verwendeten Begriffe und zugeordneten Bezugszeichen verwendet. In der Zeichnung bedeutet:
- Fig. 1: Eine perspektive Darstellung eines Rotors mit zwei Behälteraufnahmepaare, in denen je zwei fluidisch miteinander kommunizierende Behälter gehalten sind;
- Fig. 2: Eine perspektivische Darstellung des Zentrifugenkopfes aus der Fig. 1 ohne aufgesetzten Rotor;
- Fig. 3: Eine perspektive Darstellung zweier über einen Schlauch fluidisch kommunizierend miteinander verbundener Röhrchen zum Einsetzen in die Zentrifuge;
- Fig. 4: Eine perspektive Darstellung des Schlauchs aus Fig. 3;
- Fig. 5: Eine Explosionsdarstellung als auch eine Darstellung im zusammengebauten Zustand eines Sekundärröhrchens.

In der nachfolgenden Beschreibung und in den Figuren werden zur Vermeidung von Wiederholungen gleiche Bauteile und Komponenten mit gleichen Bezugszeichen gekennzeichnet, sofern keine weitere Differenzierung erforderlich oder sinnvoll ist.

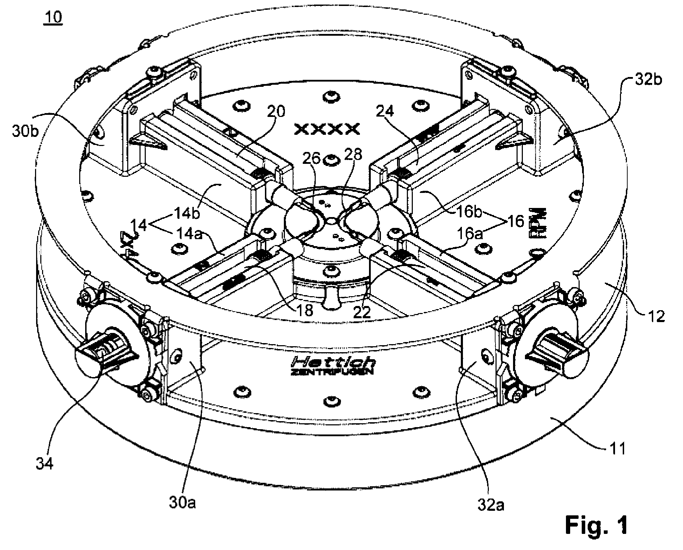

Fig. 1 zeigt in einer perspektiven Darstellung eine insgesamt mit der Bezugsziffer 10 bezeichneten Zentrifuge. Aus Gründen der Übersichtlichkeit ist vorliegend lediglich ein Zentrifugenkopf 11 und ein auf den Zentrifugenkopf 11 aufgesetzter Rotor 12 dargestellt. Die zugehörige Regel- und/oder Steuereinheit sowie die Antriebseinheit der Zentrifuge 10 sind ebenfalls aus Gründen der Übersichtlichkeit nicht dargestellt.

Der Rotor 12 weist insgesamt vier Behälteraufnahmen 14a, 14b sowie 16a, 16b auf. Die beiden Behälteraufnahmen 14a und 14b werden nachfolgend auch vereinfacht als Behälteraufnahmepaar 14 und die Behälteraufnahmen 16a und 16b als Behälteraufnahmepaar 16 bezeichnet. In jedem Behälteraufnahmepaar 14, 16 ist jeweils ein Paar fluidisch miteinander kommunizierende Behälter in Form eines Röhrchens mit einem im Innem des Röhrchens verfahrbar angeordneten Kolben gehalten. Nämlich in Behälteraufnahmepaar 14 in Behälteraufnahme 14a ein Röhrchen 18 und in Behälteraufnahme 16a ein Sekundärröhrchen 20. Entsprechend weist Behälteraufnahmepaar 16 in der Behälteraufnahme 16a ein Röhrchen 22 und in der Behälteraufnahme 16b ein Sekundärröhrchen 24 auf. Die Röhrchen 18, 20 sowie 22, 24 sind jeweils über einen transparenten Schlauch 26, 28 fluidisch kommunizierend miteinander verbunden und bilden so ein geschlossenes System zur sterilen Prozessierung von Blutproben.

Wie Fig. 1 weiter zu entnehmen ist, sind die Röhrchen 18, 20 sowie 22, 24 jeweils in radiale Richtung ausgerichtet und die transparenten Schläuche 26,28 sind in einer zentralen Einheit geführt. Jedem Schlauch 26, 28 ist hierbei ein erster optischer Sensor zur Detektierung einer im Schlauch auftretenden Trennschicht zugeordnet. Zudem wird das Vorhandensein eines Schlauchs mittels eines jedem Schlauch 26, 28 zugeordneten, ebenfalls als optischer Sensor ausgebildeten, zweiten Sensors überwacht. Die Sensoren sind vorliegend aus Gründen der Übersichtlichkeit nicht dargestellt.

Wie Fig. 1 weiter zu entnehmen ist, ist jeder Behälteraufnahme 14a, 14b sowie 16a, 16b radial außen eine Motor- und Getriebeeinheit 30a, 30b sowie 32a, 32b zugeordnet, die jeweils über eine Schubstange mit dem in dem jeweiligen Röhrchen befindlichen Kolben in Wirkverbindung stehen. Aus Gründen der Übersichtlichkeit ist vorliegend lediglich bei der Motor- und Getriebeeinheit 30 eine Schubstange 34 dargestellt.

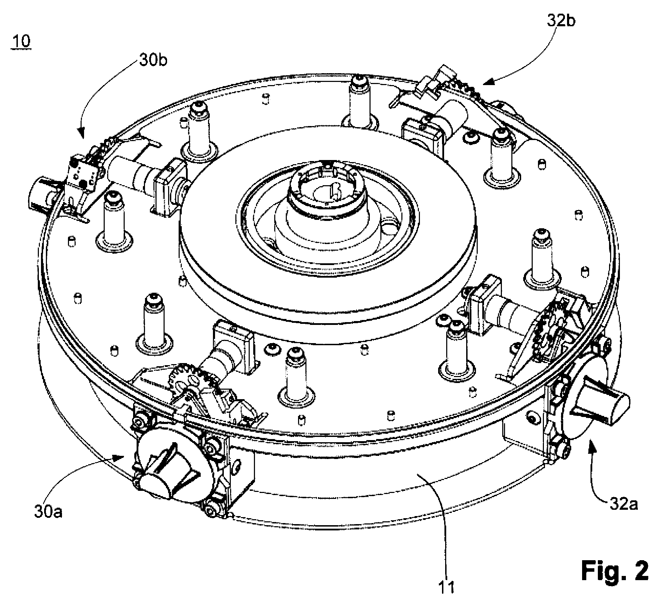

Fig. 2 zeigt den Rotorkopf 11 nach Abnahme des Rotors 12. Hierbei sind nochmals in einer Einzelheit die Motor- und Getriebeeinheiten 30a, 30b sowie 32a, 32b dargestellt.

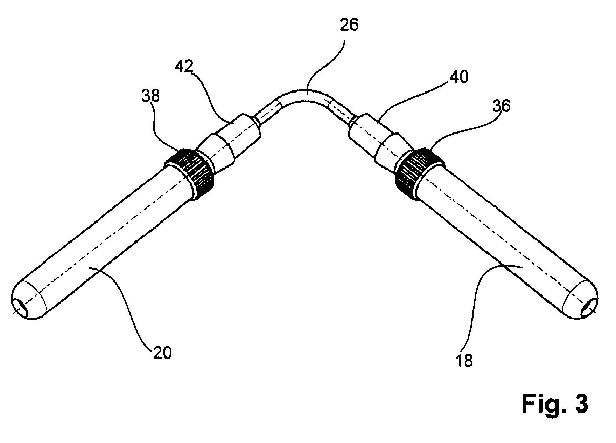

Fig. 3 zeigt in einer vergrößerten Darstellung die im ersten Behälteraufnahmepaar 14 gehaltenen Röhrchen 18 und 20. Wie Fig. 3 zu entnehmen ist, weisen die Röhrchen 18, 20 jeweils eine lösbare Verschlusskappe 36, 38 auf, während der transparente Schlauch 26 an seinen Enden mit Befestigungselemente 40, 42 versehen ist. Über die jeweils zugeordneten Elemente Verschlusskappe/Befestigungselement 36/40 bzw. 38/42 ist die fluidischen Verbindung zwischen den Röhrchen 18, 20 mittels des Schlauchs 26 auf eine einfache Art und Weise herstellbar. Während das Röhrchen 18 zur Aufnahme des Vollbluts dient, ist das Sekundärröhrchen 20 zur Aufnahme einer abgetrennten Blutkomponente vorgesehen. Der Aufbau der in zweiten Behälteraufnahmepaar 16 gehaltenen Röhrchen 22, 24 ist entsprechend.

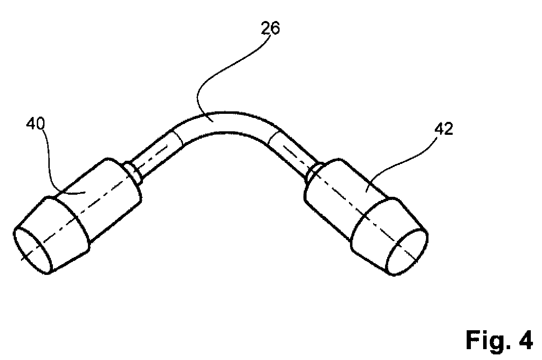

In Fig. 4 ist nochmals der transparente Schlauch 26 mit seinen zugehörigen Befestigungselementen 40, 42 dargestellt.

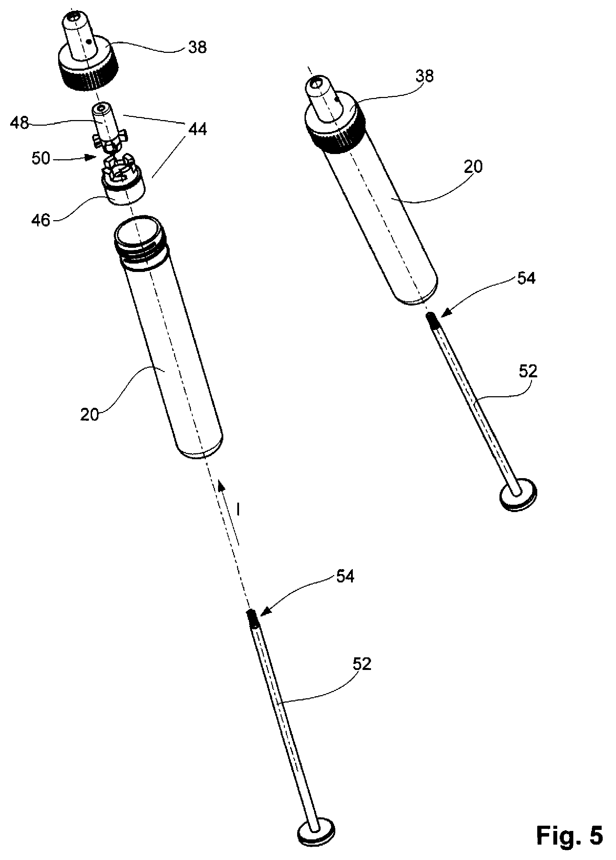

Fig. 5 zeigt das Sekundärröhrchen 20,24 einmal im montierten und einmal im demontierten Zustand. Wie der Explosionsdarstellung zu entnehmen ist, weist das Röhrchen 20,24 einen zweigeteilten Kolben 44 auf.

Der zweigeteilte Kolben 44 umfasst ein in Einschubrichtung I betrachtet hinteren Kolbenabschnitt 46 sowie einen vorderen Kolbenabschnitt 48, die mittels eines Bajonettverschlusses 50 lösbar miteinander verbindbar sind. Zudem weist der hintere Kolbenabschnitt 48 eine mit einer Dichtung verschlossene Durchgangsbohrung auf. Weiterhin ist dem Sekundärröhrchen 20 bzw. 24 eine Kolbenstange 52 zugeordnet, die im vorderen Bereich ein Außengewinde 54 aufweist.

Hierdurch ist sichergestellt, dass nach Beendigung der Zentrifugation und Entnahme des Sekundärröhrchens 20, 24 aus der Zentrifuge eine Mischung der im Sekundärröhrchen 20, 24 befindlichen Blutkomponente durchführbar ist. Hierzu wird die Kolbenstange 52 in das Sekundärröhrchen 22, 24 eingeführt und durch die im hinteren Kolbenabschnitt 46 befindliche Durchgangsbohrung hindurchgeschoben und anschließend mit den vorderen Kolbenabschnitt 48 verschraubt. Nachdem eine Verbindung zwischen Kolbenstange 52 und vorderen Kolbenelement 48 hergestellt ist, wird mittels einer weiteren Drehung der Bajonettverschluss 50 gelöst und der vordere Kolbenabschnitt 48 vom hinteren Kolbenabschnitt 46 getrennt. Mittels einer im Bezug zum hinteren Kolbenabschnitt 46 Auf- und Abbewegung des vorderen Kolbenabschnitts 48, wird die darin befindliche Blutkomponente mit dem optional vorhandenen biokompatiblen Wirkstoff manuell gemischt. Nach dem Durchmischungsvorgang wird mittels der Kolbenstange 52 der vordere Kolbenabschnitt 48 zurückgezogen und wieder mit dem hinteren Kolbenabschnitt 46 befestigt.

Mit der Zentrifuge 10 ist nun in vorteilhafter Weise die Herstellung eines hochangereicherten Thrombozytenkonzentrats Vollblut aus bzw. einer Paste aus biokompatiblen Werkstoff und Thrombozytenkonzentrat ermöglicht.

Hierzu wird ein erstes mit Vollblut gefülltes Röhrchen 18 sowie Sekundärröhrchen 20, welches optional einen biokompatiblen Werkstoff enthält, in das erste Behälteraufnahmepaar 14 eingesetzt. Ein zweites mit Vollblut gefülltes, gewichtsgleiches Röhrchen 22 und sein zugehöriges, ebenfalls optional einen biokompatiblen Werkstoff enthaltenes, Sekundärröhrchen 24 werden in das zweite Behälteraufnahmenpaar 16 eingesetzt.

Aufgrund des Schlaucherkennungssensor würde ein nicht eingesetztes zweites Behälterpaar und somit eine Unwucht vorzeitig erkannt werden.

Nachdem die Röhrchen 18,20 sowie 22,24 eingesetzt sind, erfolgt ein Anlaufen der Zentrifuge 10. Der erste Zentrifugiervorgang dauert so lange, bis sich Röhrchen 18,22 Erythrozytenkonzentrat mit höherer Dichte sowie durch eine Trennschicht davon separiertes thrombozytenreiches Blutplasma ausgebildet haben.

Anschließend wird die Drehzahl der Zentrifuge 10 reduziert und die dem Röhrchen 18 bzw. dem Röhrchen 22 zugeordnete Motor- und Getriebeeinheiten 30a, 32a laufen an und bewegen über die zugeordneten Schubstangen den in den Röhrchen 18,22 befindliche Kolben. Das heißt, es erfolgt ein Auspressen des thrombozytenreichen Blutplasmas über den transparenten Schlauch 26, 28 in die zugeordneten Sekundärröhrchen 20, 24, welche wie bereits ausgeführt, optional einen biokompatiblen Werkstoff enthalten. Der Auspressvorgang dauert hierbei solange, bis in dem transparenten Schlauch 26, 28 von dem zugeordneten Sensor die Trennschicht detektiert wurde.

Anschließend wird die Drehzahl der Zentrifuge 10 wieder erhöht und es erfolgt ein zweiter Zentrifugiervorgang. Der zweite Zentrifugiervorgang dauert so lange, bis sich im Sekundärröhrchen 22, 24 ein Thrombozytenkonzentrat mit höherer Dichte sowie ein thrombozytenarmes Blutplasma ausgebildet haben.

Anschließend wird die Drehzahl wieder reduziert und die den Sekundärröhrchen 20, 24 zugeordneten Motor- und der Getriebeeinheiten 30b, 32b laufen an. Es erfolgt eine Auspressung eines vorher definierten Volumenelements aus den Sekundärröhrchen 22, 24 über den Schlauch 26, 28 in die Röhrchen 18, 20, so dass ein hochangereichertes Thrombozytenkonzentrat in den Sekundärröhrchen 22, 24 verbleibt.

Nach Abbremsen der Zentrifuge und Zurückfahren der Motor- und Getriebeeinheiten 30a, 30b, 32a und 32b können anschließen die Röhrchen 18, 20, 22, 24 entnommen werden.

Bei den Sekundärröhrchen 20, 24 wird optional in einem weiteren Verfahrensschritt jeweils eine Kolbenstange 52 eingeführt. Nach Befestigung der Kolbenstange 52 an vorderen Kolbenabschnitt 48 und Lösen des Bajonettverschlusses 50 und in der oben beschriebenen Art und Weise, wird eine Durchmischung des Sekundärröhrchen enthaltenen hochangereicherten Thrombrozytenkonzentrats mit dem optional bereits vorgelegten biokompatiblen Werkstoff zu einer homogenen Paste vorgenommen.

Anschließend erfolgt die Entnahme des hochangereicherten Thrombozytenkonzentrats bzw. der Paste aus den Sekundärröhrchen 20, 24 durch Auspressen.

### Bezugszeichenliste

- 10: Zentrifuge
- 11: Zentrifugenkopf
- 12: Rotor
- 14a: Behälteraufnahme
- 14b: Behälteraufnahme
- 16a: Behälteraufnahme
- 16b: Behälteraufnahme
- 14: erstes Behälteraufnahmepaar
- 16: zweites Behälteraufnahmepaar
- 18: Behälter/Röhrchen
- 20: Behälter/Sekundärröhrchen
- 22: Behälter/Röhrchen
- 24: Behälter/Sekundärröhrchen
- 26: transparenter Schlauch
- 28: transparenter Schlauch
- 30a: Rotor- und Getriebeeinheit
- 30b: Rotor- und Getriebeeinheit
- 32a: Rotor- und Getriebeeinheit
- 32b: Rotor- und Getriebeeinheit
- 34: Schubstange
- 36: Verschlusskappe
- 38: Verschlusskappe
- 40: Befestigungselement
- 42: Befestigungselement
- 44: zweigeteilter Kolben
- 46: hinterer Kolbenabschnitt
- 48: vorderer Kolbenabschnitt
- 50: Bajonettverschluss
- 52: Kolbenstange
- 54: Außengewinde

- I: Einschubrichtung

## Patentansprüche

1. Zentrifuge (10) zum Trennen von Vollblut in seine Blutkomponenten, umfassend
- eine Regel- und/oder Steuereinheit sowie eine mit der Regel- und/oder Steuereinheit gekoppelten Antriebseinheit;
- einen Rotor (12) mit mindestens zwei Behälteraufnahmen (14a, 14b; 16a, 16b) zur lösbaren Halterung von fluidisch miteinander kommunizierender Behälter (18, 20, 22, 24);
- mindestens einen zwischen den Behälteraufnahmen (14a, 14b; 16a, 16b) angeordneten, mit der Regel- und/oder Steuereinheit gekoppelten Sensor zur Detektierung einer Trennschicht;
- wobei den Behälteraufnahmen (14a,14b; 16a, 16b) jeweils eine mit der Regel- und/oder Steuereinheit gekoppelte Motor- und Getriebeeinheit (30a, 30b, 32a, 32b) zugeordnet ist, die jeweils über Mittel (34) mit dem in der jeweiligen Behälteraufnahme (14a, 14b; 16a, 16b) gehaltenen Behälter (18, 20, 22, 24) derart in Wirkverbindung stehen, dass eine Überführung und Rückführung von Blutkomponenten zwischen den Behältern initiierbar ist.

2. Zentrifuge nach Anspruch 1, **dadurch gekennzeichnet, dass** der Rotor (12) mindestens zwei, jeweils zwei Behälteraufnahmen (14a, 14b; 16a, 16b) aufweisende Behälteraufnahmepaare (14,16) zur lösbaren Halterung von je zwei fluidisch miteinander kommunizierender Behälter (18,20; 22; 24) aufweist, wobei zwischen jedem Behälteraufnahmepaar (14,16) ein Sensor zur Detektierung einer Trennschicht angeordnet ist.

3. Zentrifuge nach Anspruch 2, **dadurch gekennzeichnet, dass** jedem Behälteraufnahmepaar (14, 16) ein weiterer Sensor zugeordnet ist, über den eine Bestückung eines Behälteraufnahmepaares (14,16) mit Behältern (18, 20, 22, 24) detektierbar ist.

4. Zentrifuge nach Anspruch 1 oder 2, **dadurch gekennzeichnet, dass** der Sensor/die Sensoren zur Detektierung einer Trennschicht als optischer Sensor/optische Sensoren ausgebildet ist/sind.

5. Zentrifuge nach Anspruch 3, **dadurch gekennzeichnet, dass** die Sensoren zur Detektierung einer Bestückung eines Behälteraufnahmepaares (14, 16) als optische Sensoren ausgebildet sind.

6. Zentrifuge nach einem der vorgenannten Ansprüche, **dadurch gekennzeichnet, dass** der Rotor (12) eine induktive Schnittstelle zur induktiven Daten- und Energieübertragung aufweist.

7. Kombination fluidisch kommunizierender Behälter mit einer Zentrifuge nach einem der
vorgenannten Ansprüche, **dadurch gekennzeichnet, dass** die Behälter (18, 20, 22, 24) als ein geschlossenes System zur sterilen Verarbeitung von Blut von Blutproben ausgebildet sind, wobei die Behälter (18, 20, 22, 24) jeweils in Form eines Röhrchens mit einem im Inneren des Röhrchens verfahrbar angeordneten Kolben ausgebildet sind, und die Röhrchen (18, 20, 22, 24) jeweils über eine lösbar mit dem Kolben in Kontakt stehenden Schubstange (34) in Wirkverbindung mit der der jeweiligen Behälteraufnahme (14a, 14b; 16a, 16b) zugeordneten Motor- und Getriebeeinheit (30a, 30b; 32a, 32b) stehen.

8. Kombination fluidisch kommunizierender Behälter mit einer Zentrifuge nach Anspruch 7,
**dadurch gekennzeichnet, dass** bei mindestens einem Röhrchen der Kolben (44) zweiteilig, in Einschubrichtung (I) des Kolbens (44) betrachtet einen hinteren Kolbenabschnitt (46) und einen vorderen Kolbenabschnitt (48) aufweisend ausgebildet ist, wobei der vordere und hintere Kolbenabschnitt (46, 48) lösbar miteinander verbunden sind und der hintere Kolbenabschnitt (46) ein mit einer Dichtung fluiddicht verschlossene Durchgangsbohrung aufweist, so dass nach Lösen der beiden Kolbenabschnitte (46, 48) der vordere Kolbenabschnitt (48) mittels einer durch die Durchgangsbohrung im hinteren Kolbenabschnitt (46) hindurchführbaren Kolbenstange (52) relativ zum hinteren Kolbenabschnitt (46) bewegbar ist.

9. Kombination fluidisch kommunizierender Behälter mit einer Zentrifuge nach Anspruch 8,
**dadurch gekennzeichnet, dass** der hintere und vordere Kolbenabschnitt (46, 48) mittels eines Bajonettverschlusses (50) lösbar miteinander verbunden sind und dass der vordere Kolbenabschnitt (48) eine Bohrung mit einem Innengewinde und die Kolbenstange (52) in ihrem vorderen Bereich ein hierzu korrespondierend ausgebildetes Außengewinde (54) aufweist.

10. Kombination nach Anspruch 7 bis 9, **dadurch gekennzeichnet, dass** zwei Röhrchen (18, 20; 22, 24) mittels eines transparenten Schlauchs (26,28) fluidisch kommunizierend miteinander verbunden sind.

11. Kombination nach Anspruch 7 bis 9, **dadurch gekennzeichnet, dass** drei oder mehr Röhrchen mittels eines transparenten Schlauchs, der über ein Kuppelelement in zwei oder mehr, jeweils ein Absperrventil aufweisende Schlauchstränge aufgeteilt ist, fluidisch kommunizierend miteinander verbunden sind.

12. Kombination nach Anspruch 10 oder 11, **dadurch gekennzeichnet, dass** der Schlauch (26, 28) ein Ventil aufweist.

13. Kombination nach Anspruch 9 bis 12, **dadurch gekennzeichnet, dass** der Schlauch (26,28) an seinen Enden Befestigungselemente (40, 42) aufweist, über die der Schlauch (28,28) lösbar an die Verschlusskappen (36,38) der Röhrchen (18, 20,22,24) befestigbar ist.

14. Verfahren zur Gewinnung eines hochangereicherten Thrombozytenkonzentrats aus Vollblut, mit einer Zentrifuge (10) nach einem der Ansprüche 1 bis 6 und fluidisch kommunizierender Behälter (18,20; 22,24) nach einem der Ansprüche 7 bis 13, umfassend die Verfahrensschritte
- Einlegen mindestens zweier über einen Schlauch (26, 28) fluidisch miteinander verbundene Röhrchen (18, 20; 22,24) in die Behälteraufnahmen (14a, 14b; 16a; 16b), wobei das erste Röhrchen (18; 22) das Vollblut und das zweite Röhrchen (20,24) gegebenenfalls einen biokompatiblen Werkstoff enthält;
- Anlaufen der Zentrifuge auf eine erste Zentrifugendrehzahl;
- Erstes Zentrifugieren bis sich im ersten Röhrchen (18; 22) Erythrozytenkonzentrat mit höhere Dichte sowie durch eine Trennschicht davon separiertes thrombozytenreiches Blutplasma ausgebildet haben;
- Reduzieren der Zentrifugendrehzahl auf eine erste Abpressdrehzahl;
- Anlaufen der dem ersten Röhrchen (18; 22) zugeordneten und mit dem Kolben in Wirkverbindung stehenden Motor- und Getriebeeinheit (30a, 32a) und Auspressen des thrombozytenreiches Blutplasmas über den Schlauch (26, 28) in das zweite Röhrchen (20, 24) bis der Sensor die Trennschicht detektiert hat,
- Erhöhen der Drehzahl auf eine zweite Zentrifugendrehzahl;
- Zweites Zentrifugieren bis sich im zweiten Röhrchen (20, 24) ein Thrombozytenkonzentrat mit höherer Dichte sowie ein davon separiertes thrombozytenarmes Blutplasma ausgebildet haben;
- Reduzieren der Zentrifugendrehzahl auf eine zweite Abpressdrehzahl;
- Anlaufen der dem zweiten Röhrchen (22, 24) zugeordneten und mit dem Kolben in Wirkverbindung stehenden Motor- und Getriebeeinheit (30b, 32b) und Rückpressung des thrombozytenarmen Blutplasmas über den Schlauch (26, 28) in das erste Röhrchen (18,22);
- Abbremsen der Zentrifuge;
- Zurückfahren der Motor- und Getriebeeinheiten (30a, 30b, 32a, 32b);
- Herausnahme der Röhrchen (18, 20, 22, 24),
wobei mit Ausnahme der Schritte Einlegen und Entnahme der Röhrchen (18, 20, 22, 24) sämtliche Verfahrensschritte automatisiert gemäß einem vorher festlegbaren Ablaufschema durchgeführt werden.

15. Verfahren nach Anspruch 14, wobei das zweite Röhrchen (20; 24) einen zweigeteilten Kolben (44) gemäß Anspruch 10 aufweist und das zweite Röhrchen (20,24) einen biokompatiblen Werkstoff enthält, **dadurch gekennzeichnet,** nach Herausnahme des zweiten Röhrchens (20, 24) aus der Zentrifuge und vor Entnahme des im zweiten Röhrchen (20, 24) befindlichen Thrombozytenkonzentrats dieses mittels des vorderen Kolbenabschnitts (48) mit dem biokompatiblen Werkstoff durchmischt wird.

## Claims

1. Centrifuge (10) for separating whole blood into its components, comprising
- A closed-loop control unit and/or an open-loop control unit, as well as a drive unit coupled to said closed-loop control unit and/or said open-loop control unit;
- a rotor (12) with at least two container receptacles (14a, 14b; 16a, 16b) within which containers (18, 20, 22, 24) which are in fluid communication with each other are detachably mounted;
- at least one sensor which is arranged between said container receptacles (14a, 14b; 16a, 16b) and which is coupled to said closed-loop control unit and/or said open-loop control unit, for detecting a separating layer;
- an motor and gear unit (30a, 30b, 32a, 32b) associated with each container receptacle (14a, 14b; 16a, 16b) and which is coupled to said closed-loop control unit and/or said open-loop control unit, with means (34) operatively connecting said motor and gear unit to the container (18, 20, 22,24) held in the respective container receptacle (14a, 14b; 16a, 16b) so as to enable the initiation of a transfer and/or return of blood components between said containers.

2. Centrifuge according to claim 1, **characterized in that** said rotor (12) has at least two pairs of container receptacles (14, 16) which each comprise two container receptacles (14a, 14b; 16a, 16b) within which two containers (18, 20; 22; 24) that are in fluid communication with each other are detachably mounted, with a sensor for detecting a separating layer being provided between each pair of container receptacles (14,16).

3. Centrifuge according to claim 2, **characterized in that** each pair of container receptacles (14, 16) has a further sensor associated with it, which sensor can be used to detect the presence of containers (18, 20, 22, 24) in a pair of container receptacles (14, 16).

4. Centrifuge according to claims 1 or 2, **characterized in that** the one or plural sensor(s) for detecting a separating layer are designed as one or plural optical sensor(s).

5. Centrifuge according to claim 3, **characterized in that** said sensors for detecting the presence of containers in a pair of container receptacles (14,16) are designed as optical sensors.

6. Centrifuge according to one of the preceding claims, **characterized in that** said rotor (12) has an inductive interface for the inductive transmission of data and power.

7. Combination of containers which are in fluid communication with each other with a centrifuge according to one of the preceding claims, **characterized in that** said containers (18,20, 22, 24) are designed as a closed system for the sterile processing of blood from blood samples, in which each of said containers (18, 20, 22, 24) is in the form of a small tube within which a piston is movably mounted, and these small tubes (18,20,22,24) are in turn operatively connected to the motor and gear unit (30a, 30b; 32a, 32b) associated with the respective container receptacle (14a, 14b; 16a, 16b) via a push rod (34) which detachably contacts said piston.

8. Combination of containers which are in fluid communication with each other with a centrifuge according to claim 7, **characterized in that** in at least one of said small tubes, the piston (44) is of a two-part design, i.e. it is composed of a rear piston section (46) and a front piston section (48), as viewed in the direction (I) in which said piston (44) is inserted, which front and rear piston sections (46,48) are detachably connected to each other, and which rear piston section (46) has a through bore which is sealed in a fluid-tight manner by a seal so that, once the two piston sections (46,48) have been detached from each other, said front piston section (48) can be moved relative to said rear piston section (46) by means of a piston rod (52) which can be passed through the through bore provided in the rear piston section (46),

9. Combination of containers which are in fluid communication with each other with a centrifuge according to claim 8, **characterized in that** said rear and front piston sections (46,48) are detachably connected to each other via a bayonet catch (50), and that a bore with a female thread is provided within said front piston section (48) and a matching male thread (54) is provided at the front of said piston rod (52).

10. Combination according to claims 7 to 9, **characterized in that** a transparent flexible tube (26, 28) interconnects two small tubes (18, 20; 22, 24) each for fluid communication between them.

11. Combination according to claims 7 to 9, **characterized in that** a transparent flexible tube interconnects three or more small tubes each for fluid communication between them, for which purpose a coupling element subdivides said transparent flexible tube into two or more tube strands with a shut-off valve each.

12. Combination according to claims 10 or 11, **characterized in that** said flexible tube (26,28) includes a valve.

13. Combination according to claims 9 to 12, **characterized in that** mounting elements (40, 42) are provided on the ends of said flexible tube (26, 28), which elements can be used to detachably attach said flexible tube (26, 28) to the closure caps (36, 38) of said small tubes (18,20,22,24).

14. Process for extracting a highly enriched thrombocyte concentrate from whole blood by means of a centrifuge (10) according to one of claims 1 to 6 and containers (18,20; 22, 24) which are in fluid communication with each other according to one of claims 7 to 13, comprising the following steps:
- Inserting at least two small tubes (18, 20; 22, 24), which are in fluid communication with each other via a flexible tube (26, 28), into said container receptacles (14a, 14b; 16a, 16b), with said first small tube (18; 22) containing whole blood and said second small tube (20, 24) containing a biocompatible material, if necessary;
- operating said centrifuge at a first centrifuge speed;
- first centrifugation until a higher-density erythrocyte concentrate as well as platelet-rich plasma separated therefrom by a separating layer have formed in said first small tube (18; 22);
- reducing the speed of said centrifuge to a first squeezing-off speed;
- starting the motor and gear unit (30a, 32a) associated with said first small tube (18; 22) and operatively connected to said piston, and squeezing off the platelet-rich plasma into said second small tube (26, 24) via said flexible tube (26, 28) until said sensor detects the separating layer;
- increasing the speed of said centrifuge to a second centrifuge speed;
- second centrifugation until a higher-density thrombocyte concentrate as well as platelet-poor plasma separated therefrom by a separating layer have formed in said second small tube (20; 24);
- reducing the speed of said centrifuge to a second squeezing-off speed;
- starting the motor and gear unit (30b, 32b) associated with said second small tube (20, 24) and operatively connected to said piston, and squeezing back the platelet-poor plasma into said first small tube (18, 22) via said flexible tube (26, 28);
- decelerating said centrifuge;
- moving back said motor and gear units (30a, 30b, 32a, 32b);
- removing said small tubes (18, 20, 22, 24),
wherein all process steps except for the steps of inserting and removing said small tubes (18, 20,22, 24) are performed automatically according to a predefined workflow scheme.

15. Process according to claim 14 in which said second small tube (20; 24) has a two-part piston (44) according to claim 10 and said second small tube (20, 24) contains biocompatible material, **characterized in that**, after removal of said second small tube (20, 24) from the centrifuge and before removal of the thrombocyte concentrate contained in said second small tube (20, 24), said concentrate is thoroughly mixed with the biocompatible material by means of the front section of the piston (48).

## Revendications

1. Une centrifugeuse (10) pour séparer le sang entier du composants du sang, comprenant:
une unité de commande et/ou de réglage ainsi qu'une unité d'entraînement couplée à ladite unité de commande et/ou de réglage
- un rotor (12) avec au moins deux réceptacles de récipient (14a, 14b; 16a, 16b) pour fixer des récipients (18,20,22, 24) ayant une communication fluidique entre eux de façon amovible;
- au moins un capteur installé entre lesdits réceptacles de récipient (14a, 14b; 16a, 16b), couplé à ladite unité de commande et /ou de réglage afin de détecter une couche de séparation;
- une unité moteur/engrenage (30a, 30b, 32a, 32b), couplée à l'unité de commande à boucle fermée et/ou unité de commande, chacune étant attribuée aux réceptacles de récipient (14a, 14b; 16a, 16b), respectivement reliée de façon opérationnelle aux récipients (18, 20,22,24) contenus dans les réceptacles de récipient respectifs (14a, 14b; 16a, 16b) par des moyens (34), d'une manière telle qu'un transfert et un contre-transfert des composants du sang entre les récipients peut être initié.

2. La centrifugeuse selon la revendication 1, **caractérisée en ce que** le rotor (12) comprend au moins deux paires de réceptacles de récipient (14, 16), chaque paire comprenant deux réceptacles de récipient (14a, 14b; 16a, 16b) pour contenir deux récipients respectifs (18, 20; 22; 24), ayant une communication fluidique entre eux d'une façon amovible, dans lequel un capteur est installé entre chaque paire de réceptacles de récipient (14,16) afin de détecter une couche de séparation.

3. La centrifugeuse selon la revendication 2, **caractérisée en ce que** un capteur additionnel est attribué chaque paire de réceptacles de récipient (14,16), lequel capteur permet de détecter l'équipement d'une paire de réceptacles de récipient (14, 16) ayant des récipients (18, 20, 22, 24),

4. La centrifugeuse selon les revendications 1 ou 2, **caractérisée en ce que** le(s) capteur(s) pour détecter une couche de séparation est/sont conçu(s) comme un (des) capteur(s) optique(s).

5. La centrifugeuse selon la revendication 3, **caractérisée en ce que** les capteurs pour détecter l'installation d'une paire de réceptacles de récipient (14, 16) sont conçus comme des capteurs optiques.

6. La centrifugeuse selon une des revendications précédentes, **caractérisée en ce que** le rotor (12) comprend une interface inductive pour transférer des données et de l'énergie d'une façon inductive.

7. Un ensemble de récipients étant en communication fluidique avec une centrifugeuse selon une des revendications précédentes, **caractérisé en ce que** les récipients (18, 20, 22,24) sont conçus comme un système fermé pour traiter d'une manière stérile du sang à partir d'échantillons de sang, dans lequel chaque récipient (18,20,22, 24) est conçu sous forme d'un tubule comprenant à son intérieur un piston disposé d'une manière déplaçable, et chaque tubule (18, 20, 22, 24) est relié d'une façon opérationnelle à l'unité moteur/engrenage (30a, 30b; 32a, 32b), attribuée au réceptacle de récipient respectif (14a, 14b; 16a, 16b) à l'aide d'une tige poussoir (34) qui est en contact avec le piston d'une façon amovible.

8. L'ensemble de récipients étant en communication fluidique avec une centrifugeuse selon la revendication 7, **caractérisé en ce que** le piston (44) dans un moins un tubule est conçu en deux parties, comprenant une portion postérieure du piston (46) et une portion avant du piston (48) dans le sens d'insertion (I) du piston (44), dans lequel la portion avant du piston et la portion postérieure du piston (46, 48) sont reliées entre elles de façon amovible, et la portion postérieure du piston (46) comprend un alésage de passage fermé à l'aide d'un joint d'une manière étanche au fluide, de façon à que la portion avant du piston (48) est mobile par rapport à la portion postérieure du piston (46) à l'aide d'une tige de piston (52) qui peux passer à travers le trou de passage dans la portion postérieure du piston (46) après avoir détaché les deux portions du piston (46, 48).

9. L'ensemble des récipients étant en communication fluidique avec une centrifugeuse selon la revendication 8, **caractérisé en ce que** la portion postérieure et avant du piston (46, 48) sont reliées entre elles de façon amovible à l'aide d'une fermeture à baïonnette (50) et que la portion avant du piston (48) comprend un alésage avec un filetage intérieur et la tige du piston (52) comprend dans sa zone avant un filetage extérieur (54) conçus de façon correspondante.

10. L'ensemble selon les revendications 7 à 9, **caractérisé en ce que** deux tubules (18, 20; 22, 24) communiquent entre eux d'une façon fluidique à l'aide d'un tuyau transparent (26, 28).

11. L'ensemble selon les revendications 7 à 9, **caractérisé en ce que** trois ou plusieurs tubules communiquent entre eux de façon fluidique à l'aide d'un tuyau transparent, lequel est divisé en deux ou plusieurs conduites de tuyau à l'aide d'un élément de couplage, chacun de conduites comprenant une vanne de fermeture.

12. L'ensemble selon les revendications 10 ou 11, **caractérisé en ce que** le tuyau (26,28) comprend une vanne.

13. L'ensemble selon les revendications 9 à 12, **caractérisé en ce que** le tuyau (26, 28) comprend des éléments de fixation (49,42) à ses extrémités, par lesquels le tuyau (28,28) peut être fixé de façon amovible sur les capuchons de fermeture (36, 38) des tubules (18, 20, 22, 24).

14. Un procédé pour l'obtention d'un concentré très riche en plaquettes à partir du sang entier avec une centrifugeuse (10) selon les revendications 1 à 6 et des récipients (18, 20; 22,24) ayant une communication fluidique selon les revendications 7 à 13, comprenant les étapes de procédé
- l'insertion d'au moins deux tubules (18, 20; 22, 24) ayant une communication fluidique entre eux à l'aide d'un tuyau (26, 28) dans des réceptacles de récipient (14a, 14b; 16a, 16b), dans lequel le premier tubule (18; 22) contient le sang entier et le deuxième tubule (20,24) contient une substance biocompatible, le cas échéant;
- le démarrage de la centrifugeuse à une première vitesse de rotation de centrifugation;
- une première centrifugation jusqu'au développement d'un concentré d'érythrocytes avec une concentration plus élevée, et d'un plasma sanguin riche en thrombocytes, séparés par une couche de séparation venant dudit concentré d'érythrocytes;
- la réduction de la vitesse de rotation de centrifugation à une première vitesse de rotation de compression,
- le démarrage de l'unité moteur/engrenage (30a, 32a), attribuée au premier tubule (18; 22) et reliée de façon opérationnelle au piston, et transmission du plasma sanguin riche en thrombocytes à travers le tuyau (26, 28) vers le deuxième tubule (20, 24) jusqu'à que le capteur ait détecté la couche de séparation,
- l'augmentation de la vitesse de rotation à une deuxième vitesse de rotation de centrifugation;
- une deuxième centrifugation jusqu'au développement, dans le deuxième tubule (20-24), d'un concentré de thrombocytes avec une concentration plus élevée, et du plasma sanguin pauvre en thrombocytes séparé de celui-ci;
- la réduction de la vitesse de rotation de centrifugation à une deuxième vitesse de rotation de compression;
- le démarrage de l'unité moteur/engrenage (30a, 32a), attribuée au deuxième tubule (22, 24) et reliée opérationnellement au piston, ainsi que la contre-compression du plasma sanguin pauvre en thrombocytes vers le premier tubule (18, 22) à travers le tuyau (26, 28);
- la décélération de la centrifugeuse;
- la décélération des unités moteur/engrenage (30a, 30b, 32a, 32b);
- l'enlèvement des tubules (18,20,22, 24),
dans lequel toutes les étapes de procédé sont exécutées d'une façon automatisée, selon un schéma de déroulement prédéterminé, à l'exception des étapes d'insertion et enlèvement des tubules (18, 20, 22, 24).

15. Le procédé selon la revendication 14, dans lequel le deuxième tubule (20; 24) comprend un piston en deux parties (44) selon la revendication 10, et le deuxième tubule (20, 24) contient une substance biocompatible, **caractérisé en ce que** le concentré de thrombocytes est mélangé avec ladite substance biocompatible à l'aide de la portion avant du piston (48) après l'enlèvement du deuxième tubule (20,24) de la centrifugeuse et avant l'extraction dudit concentré de thrombocytes qui se trouve dans le deuxième tubule (20, 24).
